# EUROPEAN PATENT APPLICATION

(11) **EP 0 821 926 A1**
(43) Date of publication of application: **04.02.1998**
(21) Application number: 96112600.0
(22) Date of filing: 03.08.1996
(51) Int. Cl.: A61F 13/58

(54) **Tape tabs for absorbent articles**

(71) Applicant: THE PROCTER & GAMBLE COMPANY, Cincinnati, Ohio 45202 (US)
(72) Inventor: Palumbo, Gianfranco, 61352 Bad Homburg (DE)
(74) Representative: Canonici, Jean-Jacques

(57) **Abstract**

A flexible tape tab (10) of plastic material wherein the said flexible tape tab (10) comprises a portion that is rounded in cross section characterised in that the said portion is a gripping portion (15), the said gripping portion (15) comprises a melted zone of the said plastic material and the said gripping portion (15) is thicker than the said flexible tape tab (10) before the said flexible tape tab (10) is rounded.

## Description

### Field of the invention

This invention relates to a flexible tape tab that has both a rounded and thickened gripping portion for use in an absorbent article.

### Background of the invention

It is conventional to use flexible tape tabs for holding an absorbent article such as a diaper in place. In use, the side regions overlap one another at the waist region of the wearer and are fastened to the backsheet of the waist region with flexible tape tabs. The anchoring portions of the flexible tape tabs are fixed to the backsheet of the diaper using conventional means and the gripping portions are either coated with adhesive, left partially free of adhesive, comprise mechanical fastening zones or comprise a folded over region.

Tape tabs are usually cut with a cutting device from a roll of material whose width is equal to the desired tape tab length. The tape tabs are, however, quite thin. As a result of their thinness, all the edges of the tape tabs are very sharp especially the gripping portions. The phenomenon is akin to that encountered with paper. There is therefore a risk of both the wearer and the person putting the absorbent article on the wearer being cut by the sharp edges of the tape tab. The problem is one which is known in the art but the only solutions proposed hitherto, as far as the present applicants are aware, are those described hereforth.

From WO 95/20930, a method is known of manufacturing a tape tab for use in an absorbent article; the tape tab having a rounded gripping portion. The method generally comprises the steps of cutting unitary material along parallel curved transverse cut lines to produce tape tabs in the strip material having rounded corners; doubling over the strip material onto itself to from gripping portions; and cutting the strip material along parallel cut lines which extend form the transverse cut lines to the longitudinal edge to form individual tape tabs.

A complex cutting apparatus which was specially constructed to produce an adhesive tape with a rounded, adhesive-free tape tab is known from EP 0 379 850 A1. The invention involves cutting the initial plastic material in a wave shape, locating the rounded part of the gripping portion centrally in relation to the side edges such that the corners of the gripping portion lie in an adhesive region of the tape tab.

A pressure sensitive adhesive fastening tape tab for disposable absorbent articles is disclosed in EP 0 292 970 A1. The tape tab has a anchoring and a gripping portion. In particular, the non-adhesive gripping portion has a round or curvilinear terminal edge, which preferably describes a circular arc and is formed by cutting. The gripping portion edge is designed to protect the fingers of the person putting the diaper on the infant prior to fastening.

The European patent application number 95830364.6 teaches a flexible plastic tape wherein at least one edge is rounded by the application of heat to reduce its sharpness. The thickness of the rounded edge is not greater than the thickness of the tape tab before being rounded. No mention is given to gripping portions and it is understood that the edges, in the context of the specification, preferably refer to the edges cut directly from the supply roll.

As a result of the above prior art attempts, it has been recognised by those skilled in the art that it would be desirable to provide a flexible tape tab with a more effective, user-friendly gripping portion that does not require a complex manufacturing process and that results in a reduction in the utilisation of raw materials.

It has now been discovered that the benefits of the present invention range from a flexible tape tab with a portion that is rounded and thickened in order to function as an extremely effective gripping portion; a flexible tape tab that is user-friendly and that minimizes the chances of injury to the wearer or to the person putting the absorbent article on the wearer; a flexible tape tab that can be easily detached from the backsheet on removal of the absorbent article from the wearer after use or for adjustment and reattachment of the tape tabs during use; a flexible tape tab that can be manufactured easily and a flexible tape tab that results in a reduction in the utilisation of raw materials.

### Summary of the invention

In accordance with the objects of the invention, a flexible tape tab of plastic material is provided. A portion of the flexible tape tab is rounded as is viewed in cross section. This portion is a gripping portion and comprises a melted zone of the plastic material. The gripping portion is thicker than the flexible tape tab before the flexible tape tab is rounded. In a further embodiment, the zone can be partially melted. In a preferred embodiment, the gripping portion is also rounded in plan view. Both the gripping portion and the zone are formed by the application of heat. An absorbent article can comprise at least one of the flexible tape tabs.

### Brief description of the drawings

While the specification concludes with claims particularly pointing out and distinctly claiming the invention, it is believed that the invention will be better understood from the foregoing description in conjunction with the accompanying drawing in which:
Figure 1 shows an embodiment of a flexible tape tab according to the present invention.
Figure 2 shows a preferred embodiment of a flexible tape tab according to the present invention.

### Detailed description of the invention

As used herein, the term "absorbent article" refers to devices which absorb and contain body exudates, and, more specifically, refers to devices which are placed against or in proximity to the body of the wearer to absorb and contain the various exudates discharged from the body. As used herein, the term "diaper" refers to an absorbent article generally worn by infants and sufferers of incontinence that is worn about the lower torso of the wearer. It should be understood, however, that the present invention is also applicable to other absorbent articles such as incontinence briefs and undergarments, diaper holders and liners, and the like. As used herein, the term "gripping portion" describes the part of the flexible tape tab that can easily be gripped by a user of the absorbent article.

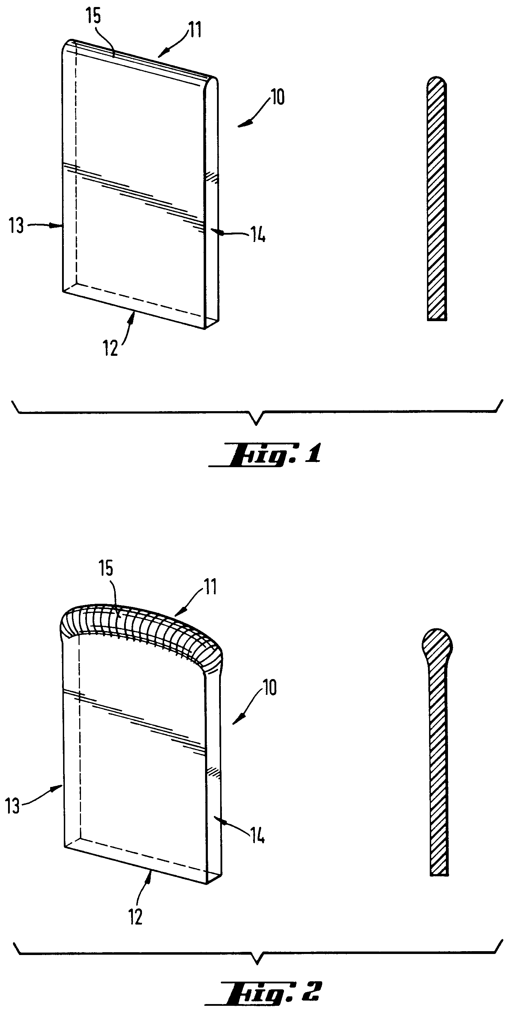

Figure 1 details an embodiment of a flexible tape tab **10** according to the present invention. The flexible tape tab **10** is made from a plastic material and is designed for use in an absorbent article. It is normal to use a strong, relatively stiff plastic material such as polypropylene. In order to ensure that the flexible tape tab **10** can function as an effective refastenable closing device, the part of the flexible tape tab **10** lying between the anchoring portion and the gripping portion **15** is covered either with adhesive, mechanical fasteners or a combination of both.

During diaper assemblage, flexible tape tabs **10** are cut from a supply roll by a cutting device, and conveyed to a point at which they are fixed in place on a diaper. As is depicted in Figure 1 and 2, the flexible tape tab **10** comprises four edges **11**, **12**, **13** and **14**. Edges **13** and **14** are the edges cut directly from the supply roll, according to the preferred method described in the background of the invention. The flexible tape tab **10** comprises a portion that is rounded in cross section - Figure 1. This portion is a gripping portion **15** and comprises a melted zone of plastic material. In a further embodiment of the invention, the zone may also be partially melted. The gripping portion for the preferred embodiment is additionally rounded in plan view - Figure 2. For the embodiment, the further embodiment and the preferred embodiment of the invention, the gripping portion **15** is thicker than the flexible tape tab **10** before the flexible tape tab **10** is rounded. The gripping portion **15** is rounded by the application of heat and the zone is similarly formed by the application of heat. In general, it is desirable that only a very small region of the flexible tape tab **10** be modified by heat, for example, a region not greater than 5 millimetres from the edge **11** of the flexible tape tab **10**, whose overall width is in the region of 30 millimetres. This is desirable both for aesthetic reasons and to avoid adversely affecting the functional characteristics of the main body of the flexible tape tab **10**.

The rounding and the formation of the melted zone or partially melted zone are done by arranging for the edge **11** of the flexible tape **10** to contact a hot metallic surface during the above-mentioned conveying process. This results in the edge **11** being melted and thereby assuming a rounded and thickened shape as viewed in cross section. Alternatively, and this is preferred, the hot metallic surface is formed by the cutting device itself, which is kept at the desired elevated temperature. One point to note is that where rounding and thickening is achieved by contact with a hot surface, the plastic material of the flexible tape tab **10** must have a sufficiently low elongational viscosity to avoid the occurrence of lagging; a phenomenon in which the hot surface pulls filaments of material outwardly thereof and gives rise to an irregular surface. A low viscosity, and/or the maintenance of the material at a sufficiently high temperature for a sufficient length of time, also assists in ensuring that surface tension effects are capable of producing the desired rounding and thickening of the edge **11**. Still more preferably, however, the rounding and thickening of the edge **11** is achieved by directing hot air at the edge **11** of the flexible tape tab **10**. For example, it has been found that air at 350 degrees celsius will produce the desired effect on a conventional polypropylene tape of a type normally used on a diaper.

Where rounding is achieved by contact with a hot surface, the temperature of the hot surface with which the flexible tape tab **10** is in contact can be different depending on the polymer nature and structure, on the crystallinity fraction and on the process speed and so on the time of contact. Similar considerations apply where hot air is used to achieve rounding and the formation of the zone.

In the case of highly crystalline polymers, for example, homopolymer polypropylene, it is useful to work in a range between 20 degrees celsius below and 50 degrees celsius above its crystalline melting point. In the case of polymers with lower crystallinity (for example, propylene/ethylene copolymers), it is more meaningful to refer to the softening point of the polymer (for example, determined by the ring and ball method according to ASTM E28-67) and work in a similar range around it.

In the various embodiments, the tape material is preferably a polypropylene copolymer containing up to **10** percent of ethylene. Such a composition results in an intrinsically softer material, which aids in a partial reduction of the sharpness of the edges and a lowering of the softening point, and facilitates the application of the present invention. More preferably, this copolymer is a random copolymer. These materials have a limited heat shrinkability, which helps the curving of the edge **11** without giving rise to macroscopic distortion of the flexible tape **10**. For example, MOPLEN EP2 C30F is a random polypropylene copolymer containing 2 percent by weight of ethylene, and is available from HIMONT. At 100 degrees celsius, it shrinks about 3 percent. Depending on the flexible tape tab **10** structure, this level of shrinkage can be useful in the present invention. In any case, the degree of heat shrinkage can be tailored to the desired level by properly formulating the polymer. The invention, however, can also be applied even where the plastic material is not particularly endowed with a high degree of heat shrinkability. In such a case, the temperature at which the edge **11** is treated needs to be sufficient to cause melting of the material, thus permitting surface tension to cause the necessary rounding.

### GLOSSARY

- 10: Flexible tape tab
- 11, 12, 13 ,14: Edges of tape tab
- 15: Gripping portion

## Claims

1. Flexible tape tab (10) of plastic material wherein said flexible tape tab (10) comprises a portion that is rounded in cross section
characterised in that
said portion is a gripping portion (15), said gripping portion (15) comprises a melted zone of said plastic material and said gripping portion (15) is thicker than said flexible tape tab (10) before said flexible tape tab (10) is rounded.

2. Flexible tape tab (10) according to claim 1 wherein said zone is partially melted.

3. Flexible tape tab (10) according to any of the preceding claims wherein said gripping portion (11) is rounded in plan view.

4. Flexible tape tab (10) according to any of the previous claims wherein said gripping portion (11) is rounded by the application of heat and said zone is similarly formed by the application of heat .

5. Flexible tape tab (10) according to any of the previous claims, wherein said plastic material essentially consists of a copolymer of propylene and a further material, the inclusion of which increases the softness of the flexible tape tab (10).

6. Flexible tape tab (10) according to claim 6, wherein said plastic material is a propylene/ethylene copolymer.

7. Flexible tape tab (10) according to claim 7, wherein said plastic material comprises up to 10 percent by weight of ethylene.

8. Flexible tape tab (10) according to claims 6, 7 and 8, wherein said copolymer is a random copolymer.

9. Absorbent article comprising at least one said flexible tape tab (10) as claimed in any of the preceding claims.
